Europäisches Patentamt

European Patent Office

Office européen des brevets

(11) Publication number:  **0 179 858 B1**

# EUROPEAN PATENT SPECIFICATION

(45) Date of publication of patent specification: **01.07.92**  (51) Int. Cl.⁵: **A61K 31/715**, A61K 33/00, A61K 33/08

(21) Application number: **85902269.1**

(22) Date of filing: **11.04.85**

(86) International application number:
**PCT/US85/00652**

(87) International publication number:
**WO 85/04806 (07.11.85 85/24)**

(54) **ANTACID COMPOSITION.**

(30) Priority: **19.04.84 US 601796**

(43) Date of publication of application:
**07.05.86 Bulletin  86/19**

(45) Publication of the grant of the patent:
**01.07.92 Bulletin  92/27**

(84) Designated Contracting States:
**AT BE CH DE FR GB IT LI LU NL SE**

(56) References cited:
**US-A- 3 857 938**
**US-A- 4 140 760**
**US-A- 4 447 417**
**US-A- 4 447 417**

**DICTIONNAIRE VIDAL, 1979, "Cahier Complémentaire", OVP, Paris, FR;**

**UNLISTED DRUGS, vol. 29, no. 5, May 1977, Chatham, New Jersey, US;**

(73) Proprietor: **RHONE-POULENC RORER INTERNATIONAL (HOLDINGS) INC.**
**40 Cape Henlopen Drive**
**Lewes, Delaware 19958(US)**

(72) Inventor: **BUEHLER, John, David**
**8407 Wood Briar Drive**
**Germantown, TN 38138(US)**
Inventor: **LUBER, Joseph, Raymond**
**3029 Edmonds Road**
**Lafayette Hill, PA 19444(US)**
Inventor: **GRIM, Wayne, Martin**
**2990 Yorkshire Drive**
**Doylestown, PA 18901(US)**

(74) Representative: **Sanderson, Laurence Andrew et al**
**SANDERSON & CO. European Patent Attorneys 34, East Stockwell Street**
**Colchester Essex CO1 1ST(GB)**

EP 0 179 858 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

UNLISTED DRUGS, vol. 26, no. 12, December 1974, Chatham, New Jersey, US;

UNLISTED DRUGS, vol. 21, no. 1, January 1969, Chatham, New Jersey, US;

UNLISTED DRUGS, vol. 16, no. 8, August 1964, Chatham, New Jersey, US;

Handbook of Nonprescription Drugs, Fifth Edition, issued January, 1977 by American Pharmaceutical Association, Washington, D.C. U.S.A., See Pages 9-10

## Description

Field of the Invention

The present invention relates to pharmaceutical compositions and particularly to compositions for use in treating gastroesophageal and gastrointestinal irritations.

Esophageal pain, commonly experienced as heartburn, is symptomatic of gastric reflux. Gastric reflux occurs when small amounts of gastric juice and/or bile acids pass into the lower part of the esophagus and cause esophageal irritation.

Typically, gastric reflux, which occurs after meals, especially large meals, is aggravated by bending over or lying down, and is a common occurence in patients having a hiatal hernia, or a weakening of the esophageal sphincter. Severe episodes of gastric reflux may inflame the esophageal mucosa and lead to the more serious condition of reflux esophagitis in which severe damage or loss of squamous epithelium of the lower part of the esophagus may occur. If esophagitis is persistent or severe, an inflammatory blockage of the esophagus may develop.

The invention relates to a composition useful in the treatment of reflux esophagitis.

Reported Developments

Persistent gastric reflux has been treated by attempting to reduce gastric volume, acidity of the gastric contents, and accelerate gastric emptying. Reduction in gastric pH is commonly effected by frequent ingestion, for example, in hourly intervals, of antacid preparations such as aluminum hydroxide gel. Other methods include the administration of drugs such as bethanechiol (Urecholine®) and metachlopramide (Reglan®), which increase the tone of the lower esophageal sphincter and accelerate gastric emptying. If these methods do not reverse the inflammatory process, surgical therapy is often recommended.

Another approach to the problem of gastric reflux comprises the administration of a preparation which forms a foam or raft which floats on the stomach contents. The foam containing antacid precedes the stomach contents into the esophagus when reflux occurs and helps to protect the mucosa from further irritation. The gelatinous foam is formed by the combination of an acid insoluble gelatinous material entrapping $CO_2$ gas. Heretofore known preparations used to create the foam comprise sodium bicarbonate and either solid compositions or liquid suspensions of alginic acid or its sodium salt. Exemplary of such prior art preprarations include the product Gaviscon® (Marion Laboratories) and compositions described in U.S. Patent No. 4,140,760.

Such known compositions contain relatively small amounts of antacid material and relatively large amounts of sodium. Accordingly, they are not particularly effective when used by patients who require a substantial adjustment of gastric pH and/or problems can be encountered when they are used by patients who should not receive an excessive amount of sodium.

Unlisted Drugs, Vol. 29, No. 5, May, 1977, discloses a tablet for use in the treatment of peptic oesophagitis. The tablet contains 350 mg of alginic acid, 105 mg of sodium bicarbonate and 100 mg of an aluminium hydroxide/magnesium carbonate mixture.

Unlisted Drugs, Vol. 16, No. 8, August, 1964, discloses a powder for use as an antacid comprising 50% alginic acid, 10% aluminium hydroxide, 2.5% magnesium trisilicate, and 17% sodium bicarbonate.

U.S. Patent Specification No. 4 140 760 relates to a pharmaceutical composition for the suppression of gastric reflux. The disclosed composition contains sodium alginate, sodium bicarbonate and calcium carbonate.

The present invention relates to a low-sodium antacid composition useful in the treatment of reflux oesophagitis prepared from magnesium alginate and an effective acid-neutralizing amount of an antacid.

Another aspect of the present invention relates to an antacid composition comprising magnesium alginate and a combined form of magnesium carbonate and aluminium hydroxide, and particularly to aqueous suspensions comprising the aforesaid ingredients.

According to the invention there is provided an antacid composition for the suppression of gastric reflux, which composition is substantially free of sodium and comprises magnesium alginate, potassium bicarbonate, and an effective acid-neutralizing amount of an antacid comprising magnesium carbonate and aluminium hydroxide, the amount of said magnesium alginate being from 8:1 to 3:1 the weight of the amount of said potassium bicarbonate, and the total amount of said magnesium carbonate and said aluminium hydroxide being from 2 to 1 times the weight of said magnesium alginate.

Magnesium alginate, one of the essential material for use in the present invention, can be prepared from alginic acid, which, like its salts such as sodium alginate, is a polymeric material composed of 1,4'

linked residues of $\alpha$-D-mannuronic acid and $\beta$-L-guluronic acid. The proportions of mannuronic to guluronic acid residues varies and depends on the brown algae source from which the alginate is extracted. Table 1 shows the composition of alginic acid obtained from various types of commercially important brown algae.

Table 1

| Mannuronic Acid (M) and Guluronic Acid (G) Composition of Alginic Acid Obtained from Commercial Brown Algae | | | | |
|---|---|---|---|---|
| Species | M Content (%) | G Content (%) | M/G Ratio | M/G Ratio Range |
| Macrocystis pyrifera | 61 | 39 | 1.56 | -- |
| Ascophyllum nodosum | 65 | 35 | 1.85(1.1) | 1.40-1.95 |
| Laminaria digitata | 59 | 41 | 1.45 | 1.40-1.60 |
| Laminaria hyperborea (stipes) | 31 | 69 | 0.45 | 0.40-1.00 |
| Ecklonia cava and Eisenia bicyclis | 62 | 38 | 1.60 | -- |

A thorough discussion of the structure and properties of alginic acid and a number of its commercially available salts is found in the trade publication of Kelco, Division of Merck and Co., Inc., entitled "Algin/hydrophilic derivatives of alginic acid for scientific water control" (second edition).

The varying composition of alginic acid and its derivatives is reflected in variations in certain of its physical properties including viscosity. Viscosity measurements of commercially available alginates using a Brookfield Model LVF Viscometer at 60 rpm with the appropriate spindle at 25°C of 1 to 2% solutions range from :0,01 Pas (10 cps) to :17 Pas (17,000 cps.)

The pharmaceutical composition according to the present invention uses the magnesium salt of alginic acid and preferably magnesium alginate exhibiting a viscosity in a 7.5 weight % solution, measured at 25°C by the Brookfield Model LVT Viscometer at 12 rpm, using spindle no. 2, of 0,01 to 1,7 Pas (10 to 1700 cps) and preferably 0,1 to 1 Pas (100 to 1000 cps).

The magnesium alginate may be prepared by a number of methods, but the preferred method comprises the reaction in aqueous media of magnesium carbonate and alginic acid, followed by the adjustment of the pH of the reaction mixture to 7.5. The preferred alginic acid used to prepare the magnesium salt comprises a polymer chain of mannuronic acid and guluronic acid segments in a ratio of mannuronic to guluronic acid of 0.4:1 to 2:1, and most preferably 0.4:1 to 1:1. In terms of weight percent, it is preferred that the alginic acid comprise 28 to 35 weight % of mannuronic acid and 65 to 72 weight % of guluronic acid. One species of brown algae which provides the most preferred source of alginic acid is

Laminaria hyperborea.

The pharmaceutical composition according to the present invention includes an antacid material. The antacid material is preferably present in an amount at least sufficient to neutralize excess gastric acid present in the stomach. The weight ratio of antacid material to magnesium alginate ranges from 1:1 to 2:1. Exemplary antacid materials include magnesium hydroxide, aluminum hydroxide, magnesium carbonate, magnesium trisilicate, magaldrate, and mixtures thereof.

The preferred antacid material comprises magnesium carbonate and aluminum hydroxide as separate ingredients and/or in a combined form. This includes codried powders of magnesium carbonate and aluminum hydroxide, and compounds such as the hydrotalcites having a formula $(Al)_w(Mg)_x(OH)_y(CO_3)_2$ and described in U.S Patent No. 4,351,814, Preferred combined forms of material include $Al(OH)_3$ in an amount of 30 to 40 wt % (as $Al_2O_3$), and $MgCO_3$ in an amount of 10 to 20% (as MgO). A preferred composition is sold by Société Des Produits Chimiques Alumineux (SPCA) and contains 35% $Al_2O_3$ and 12% MgO.

The pharmaceutical composition of the present invention can be used in tablets, powders or liquids. The liquid compositions are preferably aqueous suspensions in which the amount of antacid material provides an acid neutralizing capacity of 1 to 3 mEq/ml of suspension. The tablet compositions include an amount of antacid material in each tablet which provides an acid neutralizing capacity equal to 5 to 10 ml of said aqueous suspension.

The pharmaceutical compositions of the present invention are substantially sodium free, that is, sodium is present in no more than 10 mg per dosage amount or 10 mg per 10 ml of the aqueous suspension of the present invention.

4

Certain embodiments of the present invention may include a material which produces a nontoxic gas when contacted with aqueous acid such as gastric acid. The gas-producing material may also function as the antacid material or be a separate ingredient in the composition. The preferred gas-producing material is potassium bicarbonate.

The compositions of the present invention which include a gas-producing material form a gas, after ingestion, as a result of reacting with gastric acid in the stomach. The gas is trapped in the alginic acid gel formed by the composition thereby creating a gelatinous foamy mass of lower bulk density than the gastric contents. The gelatinous mass floats to the surface of the gastric contents and forms a physical barrier to gastric reflux, and precedes stomach contents into the esophagus.

The gas-producing material is present in an amount so as to provide an adequate volume of gas to float the alginic acid gel formed when the magnesium alginate composition is contacted with the gastric contents, and most preferably in an amount equal to one-eighth to one-third the weight of magnesium alginate. It will be understood that the rigidity, strength and thickness of the foamy mass formed in contact with gastric acid will depend upon the ratio of bicarbonate to magnesium alginate, and upon the viscosity of the magnesium alginate.

A special embodiment of the present invention comprises a foamy mass forming composition including the gas producing material and a mixture of two or more viscosity grades of magnesium alginate. The use of two or more viscosity grades of the alginate provides for a stable, well-knitted raft. A preferred composition comprises three parts of a high viscosity magnesium alginate such as a viscosity of between 0,9 and 1,4 Pas (900 and 1400 cps) (7.5 weight percent solution) and two parts of low viscosity magnesium alginate such as viscosity of between 0,08 and 0,15 Pas (80 to 150 cps) (7.5 weight percent solution). The combination of the high and low viscosity grades results in a mixture of short and long alginate polymer chains and a viscosity as measured by a 5 weight percent solution of between 0,1 and 0,25 Pas (100 to 250 cps) and preferably 0,2 Pas (200 cps).

Another aspect of the present invention relates to the nature of the antacid material used in an aqueous suspension of the composition. By way of background, known floating antacid compositions include relatively small amounts of antacid material in amounts which can only neutralize a limited amount of acid and would not substantially change the gastric pH. Compositions of the present invention may include an amount of antacid material which can neutralize excess gastric acid present in the stomach. Suspensions of the type here involved should be stable for relatively long periods of time, such as one to two years, or at least be capable of being reconstituted by agitation subsequent to separation. In dilute suspensions, the coating-out of a "suspendant" is a minor problem but in more concentrated compositions it can be a major problem. Furthermore, under certain conditions, aqueous suspensions including two antacids such as magnesium carbonate and aluminum hydroxide tend to interact and selectively separate one suspendant, such as magnesium carbonate. The antacid material is preferably present in the composition in a form which minimizes the interaction.

Both of the foregoing problems can be inhibited or deterred according to the present invention by the use of a suspension stabilizer and the use of a combined form of magnesium carbonate and aluminum hydroxide. The suspension stabilizer is included in an amount which is effective to maintain the antacid material in suspension. The choice of stabilizer will depend on various factors, including the amount and viscosity grade of the magnesium alginate used in the composition and the amount, density and particle size of the antacid material. Preferably, the aqueous supensions contain from 0.1 to 1.5% weight/volume of any pharmaceutically acceptable stabilizer which preferably does not contain sodium. Exemplary suspension stabilizers include tragacanth, guar gum, avicel, solka-floc, pectin, pregelatinized potato starch, calcium carbonate, calcium saccharin, citric acid, or hydroxypropylmethylcellulose.

It is preferred that the aqueous suspensions of the present invention have a long shelf life and not be subject to deterioration by microorganisms. Consequently the liquid compositions should contain a preservative. A combination of methyl and propyl p-hydroxybenzoates (methyl and propyl paraben) may be employed, for example, in an amount of 0.1% and 0.05% weight/volume, respectively. Antioxidants may also be included in the suspension to prevent discoloration over time.

The pharmaceutical compositions of the present invention may also include one or more of a coloring, sweetening or flavoring agent.

Tablet compositions according to the present invention may include binding agents and other ingredients known in the art to facilitate mixing, compressing, improved palatability and long term stability of the tablet.

A preferred composition according to the present invention comprises:

magnesium alginate comprising 28 to 35 weight % mannuronic acid and 65 to 72 weight % guluronic acid;

EP 0 179 858 B1

an effective acid-neutralizing amount of an antacid which comprises a first portion of magnesium carbonate, and a second portion of magnesium carbonate present in combined form with aluminum hydroxide in an amount, measured as MgO, of 5 to 20 weight %;

a gas-evolving material capable of producing a non-toxic gas when contacted with aqueous acid and in an amount such that the weight ratio of said magnesium alginate to said gas-evolving material is 3:1 to 8:1;

wherein the weight ratio of said magnesium alginate to said antacid is one-half to one.

A most preferred composition comprises said first portion of magnesium carbonate and said combined form of antacid material present in the composition in a ratio of 1:1 to 3:4.

A preferred aqueous suspension includes an effective amount of suspension stabilizer as described above and exhibits an acid-neutralizing capacity of 1 to 3 mEq/ml and a viscosity of 0,1 to 0,3 Pas (100 to 300 centipoise).

A particularly preferred suspension according to the present invention comprises 30 to 90 mg/ml of magnesium alginate, 10 to 50 mg/ml of magnesium carbonate, 10 to 50 mg/ml of a combined form of aluminum hydroxide and magnesium carbonate and 2 to 20 mg/ml of potassium bicarbonate.

The present invention is illustrated by the following examples.

Example 1

The following suspension formulation is prepared using an amount of water such that each 5 ml dosage amount of suspension includes the indicated amounts of ingredients.

|  | mg/5 ml |
|---|---|
| Magnesium Alginate (0,2 Pas (200 cps)/5% w/v solution) | 250 |
| Magnesium Carbonate USP | 160 |
| Aluminum Hydroxide-Magnesium Carbonate Gel (coprecipitated) | 180 |
| Potassium Bicarbonate USP | 50 |
| Methyl Paraben USP | 5 |
| Propyl Paraben USP | 2.5 |
| Sodium Saccharin NF | 1.4 |
| Sorbitol USP | 100 |
| Flavor | q.s. |

The resulting product has a smooth consistency, is pourable and is physically and chemically stable over a period of six months at ambient temperature. The suspension has an acid neutralizing capacity of about 8.2 mEq/5ml (about 1.6 mEq/ml) and a sodium content of less than 4 mg per 5 ml.

Examples 2 through 7 describe other suspension formulations according to the present invention.

Example 2

|  | mg/5ml |
|---|---|
| Magnesium Alginate (0,2 Pas (200 cps)/5% w/v solution | 250 |
| Aluminum Hydroxide (wet gel) USP | 336 (dried basis) |
| Potassium Bicarbonate USP | 100 |
| Methyl Paraben USP | 5 |
| Propyl Paraben USP | 2.5 |
| Sodium Saccharin NF | 1.4 |
| Sorbitol USP | 100 |
| Flavor | q.s. |

Example 3

6

|  | mg/5ml |
|---|---|
| Magnesium Alginate (0,2 Pas (200 cps)/5%/w/v solution | 250 |
| Magnesium Carbonate USP | 160 |
| aluminum Hydroxide (dried gel) USP | 180 |
| Potassium Bicarbonate USP | 50 |
| Methyl Paraben USP | 5 |
| Propyl Paraben USP | 2.5 |
| Sodium Saccharin NF | 1.4 |
| Sorbitol USP | 100 |
| Flavor | q.s. |

Example 4

|  | mg/5ml |
|---|---|
| Magnesium Alginate (0,2 Pas (200 cps)/5% w/v solution) | 250 |
| Hydrotalcite | 350 |
| Potassium Bicarbonate USP | 50 |
| Methyl Paraben USP | 5 |
| Propyl Paraben USP | 2.5 |
| Sodium Saccharin NF | 1.4 |
| Sorbitol USP | 100 |
| Flavor | q.s. |

Example 5

|  | mg/5ml |
|---|---|
| Magnesium Alginate (0,2 Pas (200 cps)/5% w/v solution) | 250 |
| Magnesium carbonate USP | 160 |
| Aluminum Hydroxide Magnesium Carbonate Gel (coprecipitated) | 180 |
| Potassium Bicarbonate USP | 50 |
| Hydroxypropylmethylcellulose USP | 25 |
| Methyl Paraben USP | 5 |
| Propyl Paraben USP | 2.5 |
| Sodium Saccharin NF | 1.4 |
| Sorbitol USP | 100 |
| Flavor | q.s. |

Example 6

|  | mg/5ml |
|---|---|
| Magnesium Alginate (0,2 Pas (200 cps)/5% w/v solution) | 250 |
| Magnesium Carbonate NF | 160 |
| Aluminum Hydroxide-Magnesium Carbonate Gel (coprecipitated) | 180 |
| Potassium Bicaronate USP | 50 |
| Tween 80 USP | 2.5 |
| Methyl Paraben USP | 5 |
| Propyl Paraben USP | 2.5 |
| Sodium Saccharin NF | 1.4 |
| Sorbitol USP | 100 |
| Flavor | q.s. |

Example 7

|  | mg/5ml |
|---|---|
| Magnesium Alginate (0,2 Pas (200 cps)/5% w/v solution) | 250 |
| Aluminum Hydroxide gel USP | 336 (dried basis) |
| Potassium Bicarbonate USP | 100 |
| Hydroxy Propyl Methylcellulose USP | 25 |
| Methyl Paraben USP | 5 |
| Propyl Paraben USP | 2.5 |
| Sodium Saccharin NF | 1.4 |
| Sorbitol USP | 100 |
| Flavor | q.s. |

The following examples relate to tablet formulations.

Example 8

Half strength tablet formulations are prepared from the following formulations.

|  | mg/tablet |
|---|---|
| Magnesium Alginate (1-1,7 Pas (1000-1700 cps)/7.5% w/v solution) | 250 |
| Magnesium Carbonate USP | 160 |
| Codried Aluminum Hydroxide - Magnesium Carbonate | 180 |
| Potassium Bicarbonate Powder USP | 50 |
| Compressible sugar NF | 750 |
| Pregelatinized starch USP | 55 |
| Magnesium Stearate NF | 14 |
| Flavor powder | q.s. |

Example 9

|  | mg/tablet |
|---|---|
| Magnesium Alginate (1-1,7 Pas (1000-1700 cps)/7.5% w/v solution) | 250 |
| Magnesium Carbonate USP | 160 |
| Aluminum Hydroxide - Magnesium Carbonate Gel (coprecipitated) | 180 |
| Compressible sugar NF | 750 |
| Potassium Bicarbonate Powder USP | 50 |
| Magnesium Stearate NF | 14 |
| Flavor powders | q.s. |

Example 10

|  | mg/tablet |
|---|---|
| Magnesium Alginate (1-1,7 Pas (1000-1700 cps)/7.5% w/v solution) | 250 |
| Aluminum Hydroxide USP | 336 |
| Potassium Bicarbonate powder USP | 50 |
| Compressible sugar NF | 750 |
| Magnesium Stearate NF | 14 |
| Pregelatinized starch USP | 55 |
| Flavor powders | q.s. |

Example 11

|  | mg/tablet |
|---|---|
| Magnesium Alginate (1-1,7 Pas (1000-1700 cps)/7.5% w/v solution) | 250 |
| Aluminum Hydroxide USP | 180 |
| Magnesium Hydroxide USP | 110 |
| Potassium Bicarbonate USP | 50 |
| Compressible sugar NF | 750 |
| Pregelatinised starch USP | 55 |
| Magnesium Stearate NF | 14 |
| Flavor powders | q.s. |

Example 12

|  | mg/tablet |
|---|---|
| Magnesium Alginate (1-1,7 Pas (1000-1700 cps)/7.5% w/v solution) | 250 |
| Magnesium Carbonate USP | 160 |
| Aluminum Hydroxide USP | 180 |
| Compressible Sugar NF | 750 |
| Potassium Bicarbonate USP | 50 |
| Pregelatinized Starch USP | 55 |
| Magnesium Stearate NF | 14 |
| Flavor powders | q.s. |

Example 13

|  | mg/tablet |
|---|---|
| Magnesium Alginate (1-1,7 Pas (1000-1700 cps)/7.5% w/v solution) | 250 |
| Hydrotalcite | 350 |
| Compressible sugar NF | 750 |
| Potassium Bicarbonate USP | 50 |
| Pregelatinized starch USP | 55 |
| Magnesium stearate NF | 14 |
| Flavor powders | q.s. |

Example 14

A full strength tablet is prepared by doubling the amounts of ingredients in the tablet examples above.

The tablet of Example 9 is prepared by mixing the ingredients together and compressing the mixture into tablet form.

The tablet of Examples 8 and 10 through 13 is prepared as follows:

All ingredients listed above except magnesium stearate are screened to eliminate unacceptably large particles. The screened ingredients are placed in a suitable mixer and blended. The resultant mixed granulate is wetted with less than about one ml of deionized water per full strength tablet and placed on trays in an oven to dry to an appropriate moisture level. The dried granulate is milled through an appropriate screen. Magnesium stearate is screened and blended with the dried granulate. The blended mix is compressed into tablets.

A further optional ingredient for incorporation into the tablet formulation comprises a mild carboxylic acid, such as alginic acid. The acid functions to provide a sensation of fizzing in the mouth while chewing the tablet. The amount of the acid is directly dependent on the desired intensity of the fizzing sensation and increases in accordance with the increased fizzing action. If the desired fizzing action is substantial, then the amount of carbonate-containing antacid material in the tablet is also increased so as to provide a sufficient source of carbon dioxide in the mouth as well as in the stomach. The additional carbonate-containing antacid material may comprise about an acid equivalent amount of material.

**Claims**

**Claims for the following Contracting States : BE, CH, DE, FR, GB, IT, LU, NL, SE**

1. An antacid composition for the suppression of gastric reflux, which composition is substantially free of sodium and comprises magnesium alginate, potassium bicarbonate, and an effective acid-neutralizing amount of an antacid comprising magnesium carbonate and aluminum hydroxide, the amount of said magnesium alginate being from 8:1 to 3:1 the weight of the amount of said potassium bicarbonate, and the total amount of said magnesium carbonate and said aluminum hydroxide being from 2 to 1 times the weight of said magnesium alginate.

2. An aqueous suspension comprising an antacid composition according to claim 1 and having an acid-neutralizing capacity of from 1 to 3 mEg/ml and a viscosity of from 0,1 to 0,3 Pas (100 to 300 centipoise.)

3. A suspension according to claim 2 comprising:
   from 30 to 90 mg/ml of magnesium alginate;
   from 10 to 50 mg/ml of magnesium carbonate;
   from 10 to 50 mg/ml of a combined form of aluminum hydroxide and magnesium carbonate; and
   from 2 to 20 mg/ml of potassium bicarbonate.

4. An antacid composition for the suppression of gastric reflux according to claim 1 comprising magnesium alginate having a viscosity within the range of from 0,01 to 1,7 Pas (10 to 1700 cps) in a 7.5 weight % aqueous solution and a water insoluble antacid material comprising a combined form of magnesium carbonate and aluminum hydroxide.

5. An antacid composition according to claim 4, wherein said combined form is prepared by drying an aqueous slurry of magnesium carbonate and aluminum hydroxide.

6. An antacid composition according to claim 4 or claim 5, wherein:
aluminum hydroxide (as $Al_2O_3$) comprises from 25 to 45 weight of said combined form; and magnesium carbonate (as MgO) comprises from 5 to 20 weight % of said combined form.

7. An antacid composition according to any one of the preceding claims useful in the treatment of reflux esophagitis and comprising magnesium alginate prepared by combining magnesium carbonate and alginic acid in aqueous media and adjusting the pH of the resultant mixture to 7.5, and wherein the polymer chain of said alginic acid comprises mannuronic acid and guluronic acid segments in a ratio of mannuronic to guluronic acid of from 0.4:1 to 2:1.

8. An antacid composition according to claim 7, wherein said ratio is from 0.4:1 to 1:1.

9. An antacid composition according to claim 7 or claim 8, wherein mannuronic acid comprises from 28 to 35 weight % and guluronic acid comprises from 65 to 72 weight % of said alginic acid.

10. An antacid composition for the suppression of gastric reflux comprising:
magnesium alginate comprising from 28 to 35 wt % mannuronic acid and from 65 to 72 weight % guluronic acid;
an effective acid-neutralizing amount of an antacid which comprises a first portion of magnesium carbonate, and a second portion of magnesium carbonate present in combined form with aluminum hydroxide in an amount measured as MgO, of from 5 to 20 weight %;
potassium bicarbonate as a gas-evolving material capable of producing a nontoxic gas when contacted with aqueous acid and in an amount such that the weight ratio of said magnesium alginate to said gas-evolving material is from three to eight; and
wherein the weight ratio of said magnesium alginate to said antacid is from 0.5:1 to 1:1.

11. An antacid composition according to claim 10, wherein the ratio of said first portion of magnesium carbonate to said combined form is from 1:1 to 3:4.

12. An aqueous suspension comprising an antacid composition according to claim 11 and an effective amount of suspension stabilizer selected from calcium saccharin, calcium carbonate and citric acid; and having an acid neutralizing capacity of from 1 to 3 mEq/ml and a viscosity of 0,1 to 0,3 Pas (100 to 300 centipoise.)

13. A method of forming an antacid-containing foam on the surface of an aqueous acidic medium by contacting said acidic medium with an aqueous suspension of a composition according to any one of claims 1 or 4 to 11.

14. A method according to claim 13, wherein said suspension include a stabilizer selected from calcium saccharin and calcium carbonate.

15. A method according to claim 14, wherein said suspension includes citric acid.

16. A process for the preparation of a composition according to any one of the claims 1 or 4 to 11, which process comprises mixing magnesium alginate together with potassium bicarbonate and an effective acid-neutralizing amount of an antacid comprising magnesium carbonate and aluminum hydroxide, the amount of said magnesium alginate being from 8:1 to 3:1 the weight of the amount of said potassium bicarbonate, and wherein the total amount of said magnesium carbonate and said aluminum hydroxide is from 2 to 1 times the weight of said magnesium alginate.

17. A process according to claim 16 wherein the magnesium alginate is prepared by combining the magnesium carbonate and alginic acid in aqueous media and adjusting the pH of the resultant mixture to 7.5, and wherein the polymer chain of said alginic acid comprises mannuronic acid and guluronic acid segments in a ratio of mannuronic to guluronic acid of from 0.4:1 to 2:1

18. An antacid composition according to claim 1 or any one of claims 4 to 11 or an aqueous suspension according to any one of claims 2, 3 or 12 for use in the treatment of reflux esophagitis in patients who restrict their dietary intake of sodium.

19. Use of an antacid composition according to claim 1 or any one of claims 4 to 11 or an aqueous suspension according to any one of claims 2, 3 or 12 for the manufacture of a medicament for the treatment of reflux esophagitis in patients who restrict their dietary intake of sodium.

**Claims for the following Contracting State : AT**

1. A process for the preparation of an antacid composition for the suppression of gastric reflux, which composition is substantially free of sodium, said process comprising mixing magnesium alginate together with potassium bicarbonate and an effective acid-neutralizing amount of an antacid comprising magnesium carbonate and aluminum hydroxide, the amount of said magnesium alginate being from 8:1 to 3:1 the weight of the amount of said potassium bicarbonate, and wherein the total amount of said magnesium carbonate and said aluminum hydroxide is from 2 to 1 times the weight of said magnesium alginate.

2. A process according to claim 1, wherein the magnesium alginate is prepared by combining the magnesium carbonate and alginic acid in aqueous media and adjusting the pH of the resultant mixture to 7.5, and wherein the polymer chain of said alginic acid comprises mannuronic acid and guluronic acid segments in a ratio of mannuronic to guluronic acid of from 0.4:1 to 2:1.

3. A process according to claim 2, wherein said ratio is from 0.4:1 to 1:1.

4. A process according to claim 2 or claim 3, wherein mannuronic acid comprises from 28 to 35 weight % and guluronic acid comprises from 65 to 72 weight % of said alginic acid.

5. A process according to any of claims 1 to 4, wherein the product thereby obtained is an aqueous suspension having an acid-neutralizing capacity of from 1 to 3 mEq/ml and a viscosity of from 0.1 to 0.3 Pas (100 to 300 centipoises).

6. A process according to claim 5, wherein the product thereby obtained is a suspension comprising:
from 30 to 90 mg/ml of magnesium alginate;
from 10 to 50 mg/ml of magnesium carbonate;
from 10 to 50 mg/ml of a combined form of aluminum hydroxide and magnesium carbonate; and
from 2 to 20 mg/ml of potassium bicarbonate.

7. A process according to any of claims 1 to 6, wherein the product thereby obtained comprises magnesium alginate having a viscosity within the range of from 0.01 to 1.7 Pas (10 to 1700 cps) in a 75 weight % aqueous solution and a water-insoluble antacid material comprising a combined form of magnesium carbonate and aluminum hydroxide.

8. A process according to claim 7, wherein said combined form is prepared by drying an aqueous slurry of magnesium carbonate and aluminum hydroxide.

9. A process according to claim 7 or claim 8, in which the product thereby obtained is one wherein:
aluminum hydroxide (as $Al_2O_3$) comprises from 25 to 45 weight % of said combined form; and
magnesium carbonate (as MgO) comprises from 5 to 20 weight % of said combined form.

10. A process according to claim 1, in which the product thereby obtained is an antacid composition for the suppression of gastric reflux comprising:
magnesium alginate comprising from 28 to 35 weight % mannuronic acid and from 65 to 72 weight % guluronic acid;
an effective acid-neutralizing amount of an antacid which comprises a first portion of magnesium carbonate, and a second portion of magnesium carbonate present in combined form with aluminum hydroxide in an amount, measured as MgO, of from 5 to 20 weight %;
potassium bicarbonate as a gas-evolving material capable of producing a nontoxic gas when contacted with aqueous acid and in an amount such that the weight ratio of said magnesium alginate to said gas-evolving material is from three to eight;
and wherein the weight ratio of said magnesium alginate to said antacid is from 0.5:1 to 1:1.

EP 0 179 858 B1

**11.** A process according to claim 10, wherein the ratio of said first portion of magnesium carbonate to said combined form is from 1:1 to 3:4.

**12.** A process according to claim 11, wherein said antacid composition takes the form of an aqueous suspension including an effective amount of suspension stabilizer selected from calcium saccharin, calcium carbonate and citric acid; and having an acid neutralizing capacity of from 1 to 3 mEq/ml and a viscosity of 0.1 to 0.3 Pas (100 to 300 centipoises).

**13.** An antacid composition or an aqueous suspension thereof for the treatment of reflux esophagitis in patients who restrict their dietary intake of sodium, whenever prepared by a process according to any of the preceding claims.

**14.** Use of an antacid composition or an aqueous suspension thereof according to claim 13 for the manufacture of a medicament for the treatment of reflux esophagitis in patients who restrict their dietary intake of sodium.

**15.** A method of forming an antacid-containing foam on the surface of an aqueous acidic medium by contacting said acidic medium with an aqueous suspension of an antacid composition according to claim 13.

**16.** A method according to claim 15, wherein said suspension includes a stabilizer selected from calcium saccharin and calcium carbonate.

**17.** A method according to claim 16, wherein said suspension includes citric acid.

**Revendications**
**Revendications pour les Etats contractants suivants : BE, CH, DE, FR, GB, IT, LU, NL, SE**

**1.** Composition antiacide pour la suppression de la régurgitation gastrique, laquelle composition est sensiblement exempte de sodium et comprend de l'alginate de magnésium, du bicarbonate de potassium, et une quantité efficace pour la neutralisation acide d'un antiacide comprenant du carbonate de magnésium et de l'hydroxyde d'aluminium, la quantité d'alginate de magnésium étant de 8:1 jusqu'à 3:1 égale au poids de la quantité de bicarbonate de potassium, et la quantité totale du carbonate de magnésium et de l'hydroxyde d'aluminium étant de 2 à 1 fois le poids de l'alginate de magnésium.

**2.** Suspension aqueuse comprenant une composition antiacide selon la revendication 1 et ayant une capacité de neutralisation acide de 1 à 3 mEq/ml et une viscosité allant de 0,1 jusqu'à 0,3 Pas (100 à 300 centipoises).

**3.** Suspension selon la revendication 2 comprenant :
de 30 à 90 mg/ml d'alginate de magnésium ;
de 10 à 50 mg/ml de carbonate de magnésium ;
de 10 à 50 mg/ml d'une forme combinée d'hydroxyde d'aluminium et de carbonate de magnésium ; et
de 2 à 20 mg/ml de bicarbonate de potassium.

**4.** Composition antiacide pour la suppression de la régurgitation gastrique selon la revendication 1, comprenant de l'alginate de magnésium ayant une viscosité à l'intérieur de la fourchette allant de 0,01 jusqu'à 1,7 Pas (10 à 1 700 cps) dans une solution aqueuse de 7,5 % en poids et une matière antiacide insoluble dans l'eau comprenant une forme combinée de carbonate de magnésium et d'hydroxyde d'aluminium.

**5.** Composition antiacide selon la revendication 4, dans laquelle la forme combinée est préparée en séchant une bouillie aqueuse de carbonate de magnésium et d'hydroxyde d'aluminium.

**6.** Composition antiacide selon la revendication 4 ou la revendication 5, dans laquelle :
l'hydroxyde d'aluminium ($Al_2O_3$) comprend de 25 à 45 % en poids de la forme combinée ; et le carbonate de magnésium (MgO) comprend de 5 à 20 % en poids de la forme combinée.

13

**7.** Composition antiacide selon l'une quelconque des revendications précédentes utile dans le traitement de l'oesophagite à régurgitation et comprenant de l'alginate de magnésium préparée en combinant du carbonate de magnésium et de l'acide alginique dans des milieux aqueux et en ajustant le pH du mélange obtenu jusqu'à 7,5, et dans laquelle la chaîne polymère de l'acide alginique comprend des segments d'acide mannuronique et des segments d'acide guluronique dans un rapport entre l'acide mannuronique et l'acide guluronique allant de 0,4:1 jusqu'à 2:1.

**8.** Composition antiacide selon la revendication 7, dans laquelle le rapport se situe de 0,4:1 jusqu'à 1:1.

**9.** Composition antiacide selon la revendication 7 ou la revendication 8, dans laquelle l'acide mannuronique comprend de 28 à 35 % en poids et l'acide guluronique de 65 à 72 % en poids de l'acide alginique.

**10.** Composition antiacide pour la suppression de la régurgitation gastrique comprenant :
l'alginate de magnésium comprenant de 28 à 35 % en poids d'acide mannuronique et 65 jusqu'à 72 % en poids d'acide guluronique ;
une quantité efficace neutralisant l'acide d'un antiacide qui comprend une première portion de carbonate de magnésium, et une seconde portion de carbonate de magnésium présente sous forme combinée avec l'hydroxyde d'aluminium dans une quantité mesurée comme étant MgO, allant de 5 à 20 % en poids ;
du bicarbonate de potassium sous forme de matériau à dégagement gazeux capable de produire un gaz atoxique lorsqu'il est mis en contact avec l'acide aqueux et dans une quantité telle que le rapport pondéral entre l'alginate de magnésium et la matière à émission gazeuse soit de 3 à 8 ; et dans laquelle le rapport pondéral entre l'alginate de magnésium et l'antiacide est de 0,5:1 jusqu'à 1:1.

**11.** Composition antiacide selon la revendication 10, dans laquelle le rapport entre la première portion du carbonate de magnésium et la forme combinée va de 1:1 jusqu'à 3:4.

**12.** Suspension aqueuse comprenant une composition antiacide selon la revendication 1 et une quantité efficace de stabilisateur en suspension choisi parmi la saccharine de calcium, le carbonate de calcium et l'acide citrique ; et
ayant une capacité de neutralisation de l'acide allant de 1 à 3 mEq/ml et une viscosité de 0,1 jusqu'à 0,3 Pas (100 à 300 centipoises).

**13.** Procédé pour la formation d'une mousse contenant d'un antiacide sur la surface d'un milieu acide aqueux par mise en contact du milieu acide avec une suspension aqueuse d'une composition selon l'une quelconque des revendications 1 ou 4 à 11.

**14.** Procédé selon la revendication 13, dans lequel la suspension comprend un stabilisateur choisi parmi la saccharine de calcium et le carbonate de calcium.

**15.** Procédé selon la revendication 14, dans lequel la suspension comprend de l'acide citrique.

**16.** Procédé pour la préparation d'une composition selon l'une quelconque des revendications 1 ou 4 à 11, lequel procédé comprend les opérations consistant à mélanger l'alginate de magnésium conjointement avec le bicarbonate de potassium et une quantité efficace de neutralisation d'acide d'un antiacide comprenant le carbonate de magnésium et l'hydroxyde d'aluminium, la quantité de l'alginate de magnésium étant de 8:1 jusqu'à 3:1 égale au poids de la quantité du bicarbonate de potassium, et dans lequel la quantité totale du carbonate de magnésium et de l'hydroxyde d'aluminium est de 2 à 1 fois le poids de l'alginate de magnésium.

**17.** Procédé selon la revendication 16, dans lequel l'alginate de magnésium est préparé en combinant le carbonate de magnésium et l'acide alginique dans des milieux aqueux et en ajustant le pH du mélange obtenu à 7,5, et dans lequel la chaîne polymère de l'acide alginique comprend des segments d'acide mannuronique et d'acide guluronique dans un rapport entre l'acide mannuronique et l'acide guluronique allant de 0,4:1 jusqu'à 2:1.

**18.** Composition antiacide selon la revendication 1 ou l'une quelconque des revendications 4 à 11 ou une suspension aqueuse selon l'une quelconque des revendications 2, 3 ou 12 pour l'utilisation dans le traitement de l'oesophagite à régurgitation sur les patients qui sont soumis à une restriction de leur prise alimentaire de sodium.

19. Utilisation d'une composition antiacide selon la revendication 1 ou l'une quelconque des revendications 4 à 11 ou une suspension aqueuse selon l'une quelconque des revendications 2, 3 ou 12 pour la fabrication d'un médicament destiné au traitement de l'oesophagite à régurgitation chez des patients qui sont soumis à une restriction de leur prise alimentaire de sodium.

**Revendications pour l'Etat contractant suivant : AT**

1. Procédé pour la préparation d'une composition antiacide pour la suppression de la régurgitation gastrique, laquelle composition est sensiblement exempte de sodium, le procédé comprenant les étapes consistant à mélanger l'alginate de magnésium conjointement avec le bicarbonate de potassium et une quantité efficace pour la neutralisation de l'acide d'un antiacide comprenant du carbonate de magnésium et de l'hydroxyde d'aluminium, la quantité d'alginate de magnésium étant de 8:1 jusqu'à 3:1 égale au poids de la quantité du bicarbonate de potassium et dans lequel la quantité totale du carbonate de magnésium et de l'hydroxyde d'aluminium est de 2 à 1 fois le poids de l'alginate de magnésium.

2. Procédé selon la revendication 1, dans lequel l'alginate de magnésium est préparé en combinant le carbonate de magnésium et l'acide alginique dans des milieux aqueux et en ajustant le pH du mélange résultant à 7,5, et dans lequel la chaîne polymère de l'acide alginique comprend des segments d'acide mannuronique et d'acide guluronique dans un rapport entre l'acide mannuronique et l'acide guluronique allant de 0,4:1 jusqu'à 2:1.

3. Procédé selon la revendication 2, dans lequel le rapport est de 0,4:1 jusqu'à 1:1.

4. Procédé selon la revendication 2 ou la revendication 3, dans lequel l'acide mannuronique comprend de 28 à 35 % en poids et l'acide guluronique comprend de 65 à 72 % en poids de l'acide alginique.

5. Procédé selon l'une quelconque des revendications 1 à 4, dans lequel le produit ainsi obtenu est une suspension aqueuse ayant une capacité neutralisante d'acide de 1 à 3 mEq/ml et une viscosité allant de 0,1 à 0,3 Pas (100 à 300 centipoises).

6. Procédé selon la revendication 5, dans lequel le produit ainsi obtenu est une suspension comprenant :
de 30 à 90 mg/ml d'alginate de magnésium ;
de 10 à 50 mg/ml de carbonate de magnésium ;
de 10 à 50 mg/ml d'une forme combinée d'hydroxyde d'aluminium et de carbonate de magnésium ; et
de 2 à 20 mg/ml de bicarbonate de potassium.

7. Procédé selon l'une quelconque des revendications 1 à 6, dans lequel le produit ainsi obtenu comprend de l'alginate de magnésium ayant une viscosité située dans la plage allant de 0,01 jusqu'à 1,7 Pas (10 à 1 700 cps) dans une solution aqueuse à 7,5 % en poids et une matière antiacide insoluble dans l'eau comprenant une forme combinée de carbonate de magnésium et d'hydroxyde d'aluminium.

8. Procédé selon la revendication 7, dans lequel la forme combinée est préparée en séchant une bouillie aqueuse de carbonate de magnésium et d'hydroxyde d'aluminium.

9. Procédé selon la revendication 7 ou la revendication 8, dans lequel le produit ainsi obtenu est un produit dans lequel :
l'hydroxyde d'aluminium ($Al_2O_3$) comprend de 25 à 45 % en poids de la forme combinée ; et
le carbonate de magnésium (MgO) comprend de 5 à 20 % en poids de la forme combinée.

10. Procédé selon la revendication 1, dans lequel le produit ainsi obtenu est une composition antiacide pour la suppression du régurgitation gastrique comprenant :

de l'alginate de magnésium comprenant de 28 à 35 % en poids d'acide mannuronique et de 65 à 72 % en poids d'acide guluronique ;

une quantité neutralisante d'acide efficace d'un antiacide qui comprend une première portion de carbonate de magnésium et une seconde portion de carbonate de magnésium présente sous la forme combinée avec l'hydroxyde d'aluminium dans une quantité mesurée en MgO allant de 5 à 20 % en poids ;

du bicarbonate de potassium sous forme de matière à émission gazeuse capable de produire un gaz atoxique lorsqu'il est mis en contact avec l'acide aqueux et dans une quantité telle que le rapport pondéral entre l'alginate de magnésium et la matière à émission gazeuse est de 3 à 8 ;

et dans lequel le rapport pondéral entre l'alginate de magnésium et l'antiacide est de 0,5:1 jusqu'à 1:1.

11. Procédé selon la revendication 10, dans lequel le rapport entre la première portion de carbonate de magnésium et la forme combinée est de 1:1 jusqu'à 3:4.

12. Procédé selon la revendication 11, dans lequel la composition antiacide est sous la forme d'une suspension aqueuse comprenant une quantité efficace de stabilisateur de suspension choisi parmi la saccharine de calcium, le carbonate de calcium et l'acide citrique ; et

ayant une capacité neutralisante d'acide allant de 1 à 3 mEq/ml et une viscosité de 0,1 à 0,3 Pas (100 à 300 centipoises).

13. Composition antiacide ou suspension aqueuse de celle-ci pour le traitement de l'oesophagite à régurgitation chez des patients qui sont soumis à une restriction de leur prise alimentaire de sodium, préparée par un procédé selon l'une quelconque des revendications précédentes.

14. Utilisation d'une composition antiacide ou de sa suspension aqueuse selon la revendication 13 pour la fabrication d'un médicament pour le traitement de l'oesophagite à régurgitation chez des patients qui sont soumis à une restriction de leur prise alimentaire de sodium.

15. Procédé pour la formation d'une mousse contenant un antiacide à la surface d'un milieu acide aqueux en mettant en contact le milieu acide avec une suspension aqueuse d'une composition antiacide selon la revendication 13.

16. Procédé selon la revendication 15, dans lequel la suspension comprend un stabilisateur choisi parmi la saccharine de calcium et le carbonate de calcium.

17. Procédé selon la revendication 16, dans lequel la suspension comprend l'acide citrique.

**Patentansprüche**

**Patentansprüche für folgende Vertragsstaaten : BE, CH, DE, FR, GB, IT, LU, NL, SE**

1. Säureneutralisierende Zusammensetzung zur Unterdrückung von gastritischem Reflux, wobei die Zusammensetzung im wesentlichen frei von Natrium ist und Magnesiumalginat, Kaliumbicarbonat und eine wirksame, säureneutralisierende Menge eines säureneutralisierenden Mittels enthält, welches Magnesiumcarbonat und Aluminiumhydroxid beinhaltet, wobei die Menge des Magnesiumalginats von 8:1 bis 3:1 des Gewichts der Menge des Kaliumbicarbonats beträgt und die Gesamtmenge des Magnesiumcarbonats und des Aluminiumhydroxids das zwei- bis einfache Gewicht des Magnesiumalginats ist.

2. Wäßrige Suspension, die eine säureneutralisierende Zusammensetzung gemäß Anspruch 1 enthält und die eine Säureneutralisationskapazität von 1 bis 3 mEq/ml und eine Viskosität von 0,1 bis 0,3 Pas (100 bis 300 centipoise) aufweist.

3. Suspension gemäß Anspruch 2, die enthält:
   30 bis 90 mg/ml Magnesiumalginat;
   10 bis 50 mg/ml Magnesiumcarbonat;
   10 bis 50 mg/ml einer gemischten Form von Aluminiumhydroxid und Magnesiumcarbonat; und
   2 bis 20 mg Kaliumbicarbonat.

4. Säureneutralisierende Zusammensetzung zur Unterdrückung von gastritischem Reflux gemäß Anspruch 1, die Magnesiumalginat, das eine Viskosität im Bereich von 0,01 bis 1,7 Pas (10 bis 1700 cps) in einer 7,5 gew.-%igen wäßrigen Lösung besitzt, und ein wasserunlösliches säureneutralisierendes Material enthält, das eine gemischte Form von Magnesiumcarbonat und Aluminiumhydroxid beinhaltet.

5. Säureneutralisierende Zusammensetzung gemäß Anspruch 4, worin die gemischte Form durch Trocknung einer wäßrigen Aufschlämmung von Magnesiumcarbonat und Aluminiumhydroxid hergestellt wird.

6. Säureneutralisierende Zusammensetzung gemäß Anspruch 4 oder Anspruch 5, worin:
   - Aluminiumhydroxid (als $Al_2O_3$) 25 bis 45 Gew. der gemischten Form umfaßt; und
   - Magnesiumcarbonat (als MgO) 5 bis 20 Gew.-% der gemischten Form umfaßt.

7. Säureneutralisierende Zusammensetzung gemäß einem der vorangehenden Ansprüche, die zur Behandlung von Reflux-Ösophagitis geeignet ist und die Magnesiumalginat enthält, das durch Vereinigung von Magnesiumcarbonat und Alginsäure in wäßrigem Medium und Einstellung des pH-Wertes der resultierenden Mischung auf 7,5 hergestellt wird, und worin die Polymerkette der Alginsäure Mannuronsäure- und Guluronsäuresegmente im Verhältnis von Mannuronsäure und Guluronsäure von 0,4:1 bis 2:1 enthält.

8. Säureneutralisierende Zusammensetzung gemäß Anspruch 7, worin das Verhältnis 0,4:1 bis 1:1 beträgt.

9. Säureneutralisierende Zusammensetzung gemäß Anspruch 7 oder 8, worin Mannuronsäure 28 bis 35 Gew.-% und Guluronsäure 65 bis 72 Gew.-% der Alginsäure umfaßt.

10. Säureneutralisierende Zusammensetzung zur Unterdrückung von gastritischem Reflux, die enthält:
    - Magnesiumalginat, das 28 bis 35 Gew.-% Mannuronsäure und 65 bis 72 Gew.-% Guluronsäure enthält;
    - eine wirksame, säureneutralisierende Menge eines säureneutralisierenden Mittels, das einen ersten Anteil an Magnesiumcarbonat enthält und einen zweiten Anteil Magnesiumcarbonat, der in gemischter Form mit Aluminiumhydroxid vorliegt, in einem Anteil gemessen als MgO von 5 bis 20 Gew.-%;
    - Kaliumbicarbonat als gasentwickelndes Material, imstande nicht-toxisches Gas zu produzieren wenn es in Kontakt mit wäßriger Säure gebracht wird, und in einer solchen Menge, daß das Gewichtsverhältnis von Magnesiumalginat zum gasentwickelnden Material zwischen 3 und 8 liegt; und
    worin das Gewichtsverhältnis von Magnesiumalginat zum säureneutralisierenden Mittel von 0,5:1 bis 1:1 ist.

11. Säureneutralisierende Zusammensetzung gemäß Anspruch 10, worin das Verhältnis des ersten Anteils Magnesiumcarbonat zur gemischten Form von 1:1 bis 3:4 ist.

12. Wäßrige Suspension, die eine säureneutralisierende Zusammensetzung gemäß Anspruch 11 und eine wirksame Menge Suspensionstabilisierer enthält, der aus Calciumsaccharin, Calciumcarbonat und Zitronensäure ausgewählt ist; und
    die eine Säureneutralisationskapazität von 1 bis 3 mEq/ml und eine Viskosität von 0,1 bis 0,3 Pas (100 bis 300 centipoise) besitzt.

13. Verfahren zur Bildung eines ein säureneutralisierendes Mittel enthaltenden Schaums auf der Oberfläche eines wäßrigen sauren Mediums dadurch, daß das saure Medium mit einer wäßrigen Suspension einer Zusammensetzung gemäß einem der Ansprüche 1 oder 4 bis 11 in Berührung gebracht wird.

14. Verfahren gemäß Anspruch 13, worin die Suspension ein Stabilisierungsmittel enthält, das aus Calciumsaccharin oder Calciumcarbonat ausgewählt ist.

15. Verfahren gemäß Anspruch 14, worin die Suspension Zitronensäure einschließt.

16. Verfahren zur Herstellung einer Zusammensetzung gemäß einem der Ansprüche 1 oder 4 bis 11, wobei

17

das Verfahren das Mischen von Magnesiumalginat mit Kaliumbicarbonat und einer wirksamen, säureneutralisierenden Menge eines säureneutralisierenden Mittels umfaßt, welches Magnesiumcarbonat und Aluminiumhydroxyd enthält, wobei die Menge des Magnesiumalginat von 8:1 bis 3:1 des Gewichts des Anteils des Kaliumbicarbonats beträgt, und worin die gesamte Menge des Magnesiumcarbonats und des Aluminiumhydroxids das zwei- bis einfache des Gewichts des Magnesiumalginats ist.

17. Verfahren gemäß Anspruch 16, worin das Magnesiumalginat durch Vereinigung des Magnesiumcarbonats und Alginsäure in wäßrigen Medium und Einstellung des pH-Werts der resultierenden Mischung auf 7,5 hergestellt wird und worin die Polymerkette der Alginsäure Mannuronsäure- und Guluronsäuresegmente in einem Verhältnis von Mannuronsäure zur Guluronsäure von 0,4:1 bis 2:1 enthält.

18. Säureneutralisierende Zusammensetzung gemäß Anspruch 1 oder einem der Ansprüche 4 bis 11 oder eine wäßrige Suspension gemäß einem der Ansprüche 2, 3 oder 12 zur Verwendung bei der Behandlung von Reflux-Ösophagitis bei Patienten, die ihre diätetische Aufnahme von Natrium einschränken.

19. Verwendung einer säureneutralisierenden Zusammensetzung gemäß Anspruch 1 oder einem der Ansprüche 4 bis 11 oder einer wäßrigen Suspension gemäß einem der Ansprüche 2, 3 oder 12 zur Herstellung eines Medikaments zur Behandlung von Reflux-Ösophagitis bei Patienten, die ihre diätetische Aufnahme von Natrium einschränken.

**Patentansprüche für folgenden Vertragsstaat : AT**

1. Verfahren zur Herstellung einer säureneutralisierenden Zusammensetzung zur Unterdrückung von gastritischem Reflux, wobei die Zusammensetzung im wesentlichen frei von Natrium ist und das Verfahren das Mischen von Magnesiumalginat mit Kaliumbicarbonat und einer wirksamen, säureneutralisierenden Menge eines säureneutralisierenden Mittels umfaßt, welches Magnesiumcarbonat und Aluminiumhydroxid enthält, wobei die Menge des Magnesiumalginats von 8:1 bis 3:1 des Gewichts der Menge des Kaliumbicarbonat beträgt und in der die gesamte Menge des Magnesiumcarbonats und des Aluminiumhydroxids das zwei- bis einfache des Gewichts des Magnesiumalginats ist.

2. Verfahren gemäß Anspruch 1, worin das Magnesiumalginat durch Vereinigung von Magnesiumcarbonat und Alginsäure in wäßrigem Medium und Einstellen des pH-Werts der resultierenden Mischung auf 7,5 hergestellt wird und in welchem die Polymerkette der Alginsäure Mannuronsäure- und Guluronsäuresegmente im Verhältnis von Mannuronsäure zu Guluronsäure von 0,4:1 bis 2:1 enthält.

3. Verfahren gemäß Anspruch 2, worin das Verhältnis 0,4:1 bis 1:1 beträgt.

4. Verfahren gemäß Anspruch 2 oder Anspruch 3, in welchem Mannuronsäure 28 bis 35 Gew.-% und Guluronsäure 65 bis 72 Gew.-% der Alginsäure umfaßt.

5. Verfahren gemäß einem der Ansprüche 1 bis 4, in welchem das dabei erhaltene Produkt eine wäßrige Suspension ist, die eine Säureneutralisierungskapazität von 1 bis 3 mEq/ml und eine Viskosität von 0,1 bis 0,3 Pas (100 bis 300 centipoise) besitzt.

6. Verfahren gemäß Anspruch 5, in welchem das dabei erhaltene Produkt eine wäßrige Suspension ist, die enthält:
   30 bis 90 mg/ml Magnesiumalginat;
   10 bis 50 mg/ml Magnesiumcarbonat;
   10 bis 50 mg/ml einer vereinigten Form von Aluminiumhydroxid
und Magnesiumcarbonat; und
   2 bis 20 mg/ml Kaliumbicarbonat.

7. Verfahren gemäß einem der Ansprüche 1 bis 6, in welchem das dabei erhaltene Produkt Magnesiumalginat, das eine Viskosität im Bereich von 0,01 bis 1,7 Pas (10 bis 1700 cps) in einer 7,5 gew.-%igen wäßrigen Lösung besitzt, und ein wasserunlösliches, säureneutralisierendes Material enthält, das eine vereinigte Form von Magnesiumcarbonat und Aluminiumhydroxid beinhaltet.

**8.** Verfahren gemäß Anspruch 7, in welchem die vereinigte Form durch Trocknen einer wäßrigen Aufschlämmung von Magnesiumcarbonat und Aluminiumhydroxid hergestellt wird.

**9.** Verfahren gemäß Anspruch 7 oder Anspruch 8, in welchem in dem so erhaltenen Produkt:
- Alumniumhydroxid (als $Al_2O_3$) 25 bis 45 Gew.-% der vereinigten Form ausmacht; und
- Magnesiumcarbonat (als MgO) 5 bis 20 Gew.-% der vereinigten Form ausmacht.

**10.** Verfahren gemäß Anspruch 1, in welchem das dabei erhaltene Produkt eine säureneutralisierende Zusammensetzung zur Unterdrückung von gastritischem Reflux ist, die enthält:
- Magnesiumalginat, das 28 bis 35 Gew.-% Mannuronsäure und 65 bis 72 Gew.-% Guluronsäure enthält;
- eine wirksame, säureneutralisierende Menge eines säureneutralisierenden Mittels, das einen ersten Anteil Magnesiumcarbonat enthält und einen zweiten Anteil Magnesiumcarbonat, der in vereinigter Form mit Aluminiumhydroxid vorliegt, in einer Menge, gemessen als MgO, von 5 bis 20 Gew.-%;
- Natriumbicarbonat als gasentwickelndes Material, imstande nicht toxisches Gas zu produzieren, wenn es in Berührung mit wäßriger Säure gebracht wird, und in einer solchen Menge, daß das Gewichtsverhältnis von Magnesiumalginat zu gasentwickelndem Material zwischen 3 und 8 liegt;
und in welchem das Gewichtsverhältnis von Magnesiumalginat zu säureneutralisierendem Mittel zwischen 0,5:1 und 1:1 liegt.

**11.** Verfahren gemäß Anspruch 10, in welchem das Verhältnis von erstem Anteil Magnesiumcarbonat zu der vereinigten Form von 1:1 bis 3:4 beträgt.

**12.** Verfahren gemäß Anspruch 11, worin die säureneutralisierende Zusammensetzung die Form einer wäßrigen Suspension annimmt, die eine wirksame Menge eines Suspensionsstabilisierers einschließt, der aus Calciumsaccharin, Calciumcarbonat und Zitronensäure ausgewählt ist; und die eine Säureneutralisierungskapazität von 1 bis 3 mEq/ml und eine Viskosität von 0,1 bis 0,3 Pas (100 bis 300 centipoise) besitzt.

**13.** Säureneutralisierende Zusammensetzung oder eine wäßrige Suspension daraus zur Behandlung von Reflux-Ösophagitis bei Patienten, die ihre diätetische Natriumaufnahme beschränken, wenn immer diese nach einem Verfahren gemäß einem der vorhergehenden Ansprüche hergestellt ist.

**14.** Verwendung einer säureneutralisierenden Zusammensetzung oder einer wäßrigen Suspension daraus, gemäß Anspruch 13, für die Herstellung eines Medikaments zur Behandlung von Reflux-Ösophagitis bei Patienten, die ihre diätetische Natriumaufnahme beschränken.

**15.** Verfahren zur Bildung eines ein säureneutralisierendes Mittel enthaltenden Schaums auf der Oberfläche eines wäßrigen sauren Mediums dadurch, daß das saure Medium mit einer wäßrigen Suspension einer säureneutralisierenden Zusammensetzung gemäß Anspruch 13 in Berührung gebracht wird.

**16.** Verfahren gemäß Anspruch 15, worin die Suspension ein Stabilisierungsmittel einschließt, das aus Calciumsaccharin und Calciumcarbonat ausgewählt ist.

**17.** Verfahren gemäß Anspruch 16, worin die Suspension Zitronensäure einschließt.